# EUROPEAN PATENT APPLICATION

(11) **EP 1 582 599 A1**
(43) Date of publication of application: **05.10.2005**
(21) Application number: 05006128.2
(22) Date of filing: 21.03.2005
(51) Int. Cl.: C12Q 1/68, C12N 15/10

(54) **Method for purifying microbeads**

(30) Priority: 31.03.2004 JP 2004106060
(71) Applicant: TAKARA BIO INC., Shiga (JP)
(72) Inventor: Okamoto, Sachiko, Otsu-shi, Shiga (JP); Mineno, Junichi, Otsu-shi, Shiga (JP); Asada, Kiyozo, Otsu-shi, Shiga (JP); Kato, Ikunoshin, Otsu-shi, Shiga (JP)
(74) Representative: Grund, Martin

(57) **Abstract**

A method for purifying a microbead having an immobilized nucleic acid, a kit for the method, a microbead array which is an array of microbeads each having an immobilized nucleic acid, and a kit for preparing the microbead array.

## Description

### BACKGROUND OF THE INVENTION

### Field of the Invention

The present invention relates to a purification method for a microbead array technique in which a nucleic acid labeled with at least two labeling substances is used, and a microbead array prepared using the method.

### Description of Related Art

The DNA microbead array technique has been described in detail by Brenner et al. (e.g., JP-A 11-507528 (Patent Document 1); JP-A 2000-515006 (Patent Document 2); Brenner, S. et al., Proc. Natl. Acad. Sci. USA, 97:1665-1670, (2000) (Non-patent Document 1); and Brenner, S. et al., Nature Biotechnology, 18:630-634 (2000) (Non-patent Document 2)). According to the DNA microbead array technique, nucleic acids cloned in a tag vector (hereinafter referred to as target nucleic acids) are amplified using a polymerase chain reaction (PCR), tags are converted into single-stranded ones, and hybridization with anti-tags attached to beads makes a single target nucleic acid correspond to each single microbead. Then, microbeads to which target nucleic acids are bound as a result of the hybridization are sorted using a cell sorter.

In Non-Patent Document 1, Brenner et al. sequentially carry out the following enzymatic or chemical reactions: A) amplification of target DNAs cloned in a tag vector by a PCR using a biotin-labeled antisense primer (for tag) and a FAM-labeled sense primer (for target nucleic acid); B)cleavage of the amplification products with a restriction enzyme PacI; C) purification of the amplification products using a streptavidin substrate; D) conversion of tag portions into single-stranded ones using T4 DNA polymerase in the presence of dGTP; E) hybridization between microbeads and the amplification products; F) washing; and G) sorting of fluorescently labeled beads using a cell sorter.

According to the method of Brenner et al. as described above, use of a cell sorter is required for sorting hybridized microbeads in a purification step after hybridization of 160,000 cDNAs each having an attached tag to 16,700,000 microbeads. The percentage of the hybridized microbeads in the total is about 0.96%, and the number of unhybridized beads (99.04%) is 16,540,000. Then, a fast cell sorter is necessary for such sorting. Practically, sorting is carried out against 300,600,000 microbeads from 18 units each consisting of 16,700,000 microbeads. For this purpose, a long period of time is required for the sorting even if a fast cell sorter is used. Thus, a convenient sorting method has been desired.

### SUMMARY OF THE INVENTION

The main object of the present invention is to provide a means of conveniently sorting microbeads each having a bound target nucleic acid for preparation of a microbead array.

As a result of intensive studies, the present inventors have found that microbead each having a bound target nucleic acid can be sorted without the use of a cell sorter by labeling the target nucleic acids to be immobilized with at least two labeling substances in the above-mentioned procedure. Thus, the present invention has been completed. Specifically, target nucleic acids cloned in a tag vector are amplified using a sense primer labeled with at least two labeling substances such as a detectable labeling substance (e.g., FAM) and a labeling substance capable of binding to a solid phase (e.g., biotin), and an unlabeled antisense primer.

The first aspect of the present invention relates to a method for purifying a microbead having an immobilized nucleic acid as a target, the method comprising:
(a) immobilizing a nucleic acid labeled with at least two labeling substances to a microbead through a covalent bond, wherein at least one of the labeling substances is a detectable labeling substance and another labeling substance is a labeling substance capable of binding to a solid phase that is different from the microbead; and
(b) binding the microbead having the immobilized nucleic acid to the solid phase that is different from the microbead to isolate the bound microbead.

According to the first aspect, the at least two labeling substances may be placed on the same molecule of the nucleic acid, and the detectable labeling substance is exemplified by a labeling substance selected from the group consisting of fluorescent substances, chemiluminescent substances, enzymes and radioisotopes. The labeling substance capable of binding to a solid phase that is different from the microbead is exemplified by a labeling substance selected from the group consisting of biotin, avidin and haptens.

The second aspect of the present invention relates to a kit for the method for purifying a microbead having an immobilized nucleic acid of the first aspect.

The third aspect of the present invention relates to a microbead array which is an array of microbeads each having an immobilized nucleic acid, wherein the nucleic acid as a target immobilized on each microbead consists of molecules having an identical sequence, the nucleic acid is labeled with at least two labeling substances, at least one of the labeling substances is a detectable labeling substance and another labeling substance is a labeling substance capable of binding to a solid phase that is different from the microbead.

According to the third aspect, the at least two labeling substances may be placed on the same molecule of the nucleic acid, and the detectable labeling substance is exemplified by a labeling substance selected from the group consisting of fluorescent substances, chemiluminescent substances, enzymes and radioisotopes. The labeling substance capable of binding to a solid phase that is different from the microbead is exemplified by a labeling substance selected from the group consisting of biotin, avidin and haptens.

The fourth aspect of the present invention relates to a kit for preparing the microbead array of the third aspect.

Microbeads each having a bound target nucleic acid can be conveniently sorted for preparation of a microbead array according to the present invention.

### BRIEF DESCRIPTION OF THE SEVERAL VIEWS OF THE DRAWINGS

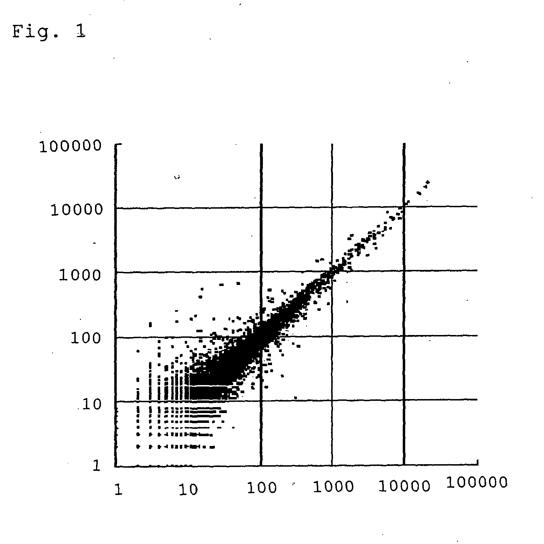

Figure 1 shows a scatter plot representing respective numbers of sequences obtained from microbeads.

### DETAILED DESCRIPTION OF THE INVENTION

The terms as used herein are defined and explained as follows. Unless otherwise defined, terms are used herein to represent the meanings as commonly understood by those skilled in the art of molecular biology, molecular genetics and the like.

"A microbead array technique" means a technique disclosed in Patent Document 1, Patent Document 2, Non-patent Document 1 or Non-patent Document 2, a technique for analyzing the expression or the structure of a gene utilizing the same, or a technique substantially equivalent thereto. Specifically, it is a technique for preparing a library of microbeads each having an immobilized target nucleic acid by placing specific target nucleic acids on specific microbeads as a result of hybridization between anti-tag sequences attached to the microbeads and tag sequences attached to the target nucleic acids. According to this technique, a target nucleic acid immobilized on each microbead consists of molecules having an identical sequence.

"Megaclone" is a technique in which nucleic acids are bound to microbeads, wherein an oligonucleotide called a tag is attached to each nucleic acid, an anti-tag which is an oligonucleotide complementary to the tag is attached to each microbead, and the bindings are accomplished by hybridization between the tags and the anti-tags. Then, a library of DNAs immobilized on microbeads in which a nucleic acid attached on each microbead consists of molecules having a single (identical) sequence can be constructed.

"A tag library" means a library of nucleic acids to be attached to microbeads according to a microbead array technique. Each clone in the tag library is a nucleic acid that contains a nucleic acid to be attached to a microbead and a tag sequence in the same molecule. The tag library is a collection of such clones.

"A tag" is an oligonucleotide bound, through a covalent bond, to a nucleic acid to be attached to a microbead according to a microbead array technique. It is used for placing a nucleic acid consisting of molecules having an identical sequence on each microbead. It is necessary that the repertoire of tags is sufficiently larger than the number of clones in the tag library.

"A single-stranded tag" means an oligonucleotide tag disclosed in Patent Document 1.

"An anti-tag" means an oligonucleotide that is completely complementary to a single-stranded tag. It is an oligonucleotide attached, though a covalent bond, to a microbead according to a microbead array technique. It has a sequence complementary to a tag. An anti-tag of a unique sequence is bound to each microbead. The repertoire of anti-tags is substantially the same as that of tags. A double-stranded product generated as a result of annealing of the single-stranded tag to the anti-tag is called a tag or a double-stranded tag.

"A tag sequence" means a sequence of an oligonucleotide used as a single-stranded tag or an anti-tag. Although there is no specific limitation concerning its structure, the tag sequence is exemplified by one containing plural subunits each consisting of an oligonucleotide of 3 to 6 bases. Although it is not intended to limit the present invention, for example, the anti-tag sequences as described in Non-patent Document 1 can be preferably used. Specifically, they are 32-base oligonucleotides each consisting of eight "words" connected each other. The "word" corresponds to the "subunit" in Patent Document 1, and is selected from the group consisting of TTAC, AATC, TACT, ATCA, ACAT, TCTA, CTTT and CAAA. The anti-tag sequences consist of 8⁸ (= about 17 million) sequences. The single-stranded tags are 32-base oligonucleotides each being complementary to the anti-tags, and consist of about 17 million sequences like the anti-tags. Furthermore, the double-stranded tags are 32-base pair double-stranded oligonucleotides generated as a result of annealing of the single-stranded tags to the anti-tags each having a sequence completely complementary to one of the single-stranded tags. The double-stranded tags also consist of about 17 million sequences.

The single-stranded tags, the anti-tags, the tags and the double-stranded tags may be oligonucleotides that are not covalently attached to other molecules. Alternatively, they may be covalently attached to other DNAs, microbeads, fluorescent substances or other molecules. Furthermore, some or all of the nucleotides may be replaced by modified nucleotides such as, without limitation, 5-methyl cytosine, 7-deaza guanine or 6-methyl adenine.

"A nucleic acid as a target" (hereinafter also referred to as a target nucleic acid) means a nucleic acid bound, or to be bound, to a microbead. It may be a DNA, an RNA or a derivative thereof. There is no specific limitation concerning the region of the target nucleic acid. It may be an entire region of a gene, or only a 5' or 3' fragment. There is no limitation concerning the origin of the target nucleic acid according to the present invention. For example, it may be derived from an animal, a plant, a eukaryotic microorganism, a prokaryotic microorganism or a virus. Also, there is no limitation concerning the method for preparing the target nucleic acid. For example, a genomic DNA, a cDNA, a synthetic DNA, a nucleic acid amplified therefrom using PCR, a restriction fragment thereof, a nucleic acid in which such a nucleic acid is cloned in a vector (e.g., a plasmid vector or a bacteriophage vector), a mixture of cloned nucleic acids, a nucleic acid in which the vector portion is removed from such a cloned nucleic acid by digestion with a restriction enzyme or the like, or a physical- or chemical-treatment product therefrom can be preferably used. In other words, any naturally occurring or artificially prepared nucleic acid can be preferably used as the target nucleic acid as long as it can be immobilized onto a microbead array used according to the present invention.

"Megasort" is a technique in which two probes labeled with distinct dyes (e.g., Cy5 and fluorescein) are used for competitive hybridization to nucleic acids on microbeads, and genes with varying expression are separated using a flow cytometer or the like.

"The MPSS method" means a technique disclosed in Patent Document 2 or Non-patent Document 2. Specifically, it is a technique in which the following steps are conducted in a flow cell: two-dimensional filling of microbeads each having an attached nucleic acid, which are prepared using a microbead array technique, into a flow cell; digestion with a type IIs restriction enzyme; ligation of an adaptor to protruding ends generated as a result of the digestion; and distinction of the nucleotide sequence of the ligated adaptor using a fluorescent probe.

As used herein, "a microbead array" means a collection of microbeads each having a target nucleic acid immobilized through a covalent bond, which is prepared using a microbead array technique. The form of the collection is not specifically limited. For example, it may be a collection of microbeads having immobilized restriction fragments from cDNAs that have been prepared from substantially all the mRNAs expressed in HepG2 cells (ATCC HB-8065).

As used herein "a nucleic acid to be immobilized" refers to a double-stranded target nucleic acid to which a single-stranded tag is covalently attached. According to the method of the present invention, the density of nucleic acids to be immobilized onto a 5-µm microbead is preferably 10³ to 10⁶ molecules per microbead, more preferably 10⁴ to 10⁵ molecules per microbead.

As used herein, "a detectable labeling substance" is a labeling substance that can be detected using a means of detection. Examples thereof include fluorescent substances, chemiluminescent substances, enzymes, radioisotopes, biotin, avidin, antigens, antibodies and haptens.

As used herein, "a labeling substance capable of binding to a solid phase" is a labeling substance that can be physically sorted. Although it is not intended to limit the present invention, for example, biotin, avidin, an antigen, an antibody or a hapten such as digoxigenin can be preferably used.

The present invention is described in detail below.

(1) Step of preparing nucleic acids containing entire or partial nucleotide sequences of genes of interest
For example, genomic nucleic acids or nucleic acids synthesized from polyA RNAs or total RNA are prepared for preparing target nucleic acids. There is no specific limitation concerning the region of the nucleic acid. It may be an entire region of a gene, or only a 5' or 3' fragment. There is no limitation concerning the origin of the nucleic acid. For example, it may be derived from an animal, a plant, a eukaryotic microorganism, a prokaryotic microorganism or a virus. Also, there is no limitation concerning the method for preparing the nucleic acid. For example, a genomic DNA, a cDNA, a synthetic DNA, a nucleic acid amplified therefrom using PCR, a restriction fragment thereof, a nucleic acid in which such a nucleic acid is cloned in a vector (e.g., a plasmid vector or a bacteriophage vector), a mixture of cloned nucleic acids, a nucleic acid in which the vector portion is removed from such a cloned nucleic acid by digestion with a restriction enzyme or the like, or a physical- or chemical-treatment product therefrom can be preferably used. In other words, any naturally occurring or artificially prepared nucleic acid can be preferably used as the target nucleic acid as long as it can be immobilized onto a microbead array used according to the present invention.

(2) Step of ligating nucleic acids to a tag vector to construct a tag library
The nucleic acids prepared in (1) are ligated to a vector having tag sequences (hereinafter referred to as a tag vector). Ones disclosed in Patent Document 1 can be used as tag sequences contained in the tag vector. In particular, ones described in Non-patent Document 1 can be preferably used. Furthermore, the tag vector desirably contains a selectable marker such as a drug resistance marker. For example, if *Escherichia coli* is to be used as a host, a drug resistance gene for ampicillin resistance, chloramphenicol resistance, kanamycin resistance, streptomycin resistance or the like can be used as a marker although it is not intended to limit the present invention.

The tag vector ligated to the nucleic acids is transferred into an appropriate host, for example, *Escherichia coli* if a vector for *Escherichia coli* is to be used. Nucleic acids can be transferred into a host according to a known method, and there is no specific limitation concerning the method. If *Escherichia coli* is to be used as a host for transformation, it is possible to use an electroporation method or a method in which competent cells are used. Transformants are cultured under selective pressure corresponding to a marker gene on a tag vector. Then, a tag library as a mixture of nucleic acids in which nucleic acid fragments are ligated to the tag vector is prepared from the cells. Upon preparation of the tag library, nucleic acids may be prepared according to a known method. For example, if a plasmid vector is used as the tag vector, an alkali-SDS method or a commercially available kit can be used.

(3) Step of preparing nucleic acids to be immobilized in which above nucleic acids are ligated to tag sequences from tag library and binding them to microbeads
The method of the present invention is characterized in that target nucleic acid portions in nucleic acids to be immobilized are labeled with plural (in particular, two) labeling substances. Various methods for labeling nucleic acids are known. For example, amino groups or thiol groups are introduced at termini, and a fluorescent dye is attached utilizing the functional groups in a common system. Alternatively, a method in which a fluorescent dye or an isotope is incorporated during amplification using PCR may be used. Thus, various methods are available.

Any labeling substance may be used as long as it can be detected. Although it is not intended to limit the present invention, for example, a fluorescent substance (a fluorescent group) such as FAM, FITC, rhodamine, ROX, JOE, TAMRA, Texas Red, Cy3 or Cy5 can be preferably used as a labeling substance. Furthermore, a labeling substance that is not a fluorescent substance may be selected from the group consisting of chemiluminescent substances, enzymes, radioisotopes, biotin, avidin, antigens, antibodies and haptens such as digoxigenin.

A detectable labeling substance and a labeling substance capable of binding to a solid phase can be selected according to the method of the present invention. Although it is not intended to limit the present invention, for example, FAM and biotin can be preferably used. Furthermore, the nucleic acid to be immobilized may be a mixture of nucleic acid molecules to be immobilized each labeled with one of the respective labeling substances. Alternatively, a single molecule of the nucleic acid to be immobilized may be labeled with plural labeling substances.

Although there is no specific limitation concerning the method for preparing the nucleic acids to be immobilized from a tag library, examples thereof include digestion with a restriction enzyme and a nucleic acid amplification reaction. In particular, a method in which target nucleic acids each containing a target nucleic acid fragment and a tag are prepared from a tag library using a nucleic acid amplification reaction (e.g., PCR) can be preferably used. In this case, a mixture of primers each labeled with a detectable labeling substance or a labeling substance capable of binding to a solid phase, or a single primer labeled with a detectable labeling substance and a labeling substance capable of binding to a solid phase may be used as a primer for target nucleic acid among the primers used in the nucleic acid amplification reaction. Unlabeled one is used as a primer for tag. It is desirable that the mixing ratio of a detectable labeling substance and a labeling substance capable of binding to a solid phase is 0.1:1 to 50:1, preferably 1:1 to 20:1. There is no specific limitation concerning the detectable labeling substance as long as it can be used for monitoring in (4) below. There is no specific limitation concerning the labeling substance capable of binding to a solid phase as long as it can be used for separation in (5) below.

According to the method of the present invention, any site may be selected for labeling as long as labeling of a nucleic acid to be immobilized is achieved. Although it is not intended to limit the present invention, for example, a method in which a labeling substance is introduced at the 5'-end of a primer to be used upon PCR-amplification of nucleic acids to be immobilized from a tag library can be preferably used. A labeled nucleotide may be incorporated upon PCR-amplification, or amplification products may be labeled by chemical modification. A labeling substance may be introduced into a base portion or a sugar portion of a nucleic acid.

For example, the tag portions of the nucleic acids are converted into single-stranded ones as follows. The tags as described in Non-patent Document 1 consist of A, T and G nucleotides, and the strands complementary to the tags consist of T, A and C nucleotides. It is possible to convert only the tag portions into single-stranded ones by allowing T4 DNA polymerase to act in the presence of dGTP optionally after removing the terminal fragments by digesting the nucleic acids with a restriction enzyme that cleaves at sites between the tags and the termini connected to the tags.

The thus obtained target nucleic acid fragments each having an attached single-stranded tag are bound to microbeads each having an attached anti-tag (hereinafter referred to as microbeads). There is no specific limitation concerning the material of the microbeads. It may vary depending on the purpose. Examples of the materials include glass, low crosslinked polystyrene, high crosslinked polystyrene, glycidal methacrylate and magnetic materials. Furthermore, there is no specific limitation concerning the size of the microbeads. For example, the size may be from 1 to 100 µm in diameter although it may vary depending on the purpose.

The microbeads can be prepared, for example, according to the method as described in Patent Document 1 or Non-patent Document 1. The target nucleic acids each having an attached single-stranded tag (the nucleic acids to be immobilized) are mixed with the microbeads, and the mixture is incubated. There is no specific limitation concerning the conditions including the composition of the incubation mixture and the temperature as long as the anti-tags on the microbeads specifically hybridize to the single-stranded tags bound to the target nucleic acid fragments under the conditions. Preferably, it is possible to use the conditions as described in Non-patent Document 1, i.e., hybridization in 500 mM NaCl, 10 mM Na phosphate, 0.01% Tween 20 and 3% dextran sulfate at 72°C for three days. "Specific hybridization" means that a tag and an anti-tag that are complementary to each other hybridize to each other, while a tag and an anti-tag that contain noncomplementary sequences hybridize only at a low frequency, or do not hybridize.

(4) Step of washing microbeads and sorting microbeads to which target nucleic acids each having an attached single-stranded tag are bound as a result of hybridization
The microbeads hybridized at 72°C for three days in step (3) above are washed. For example, if the target nucleic acids each having an attached single-stranded tag (the nucleic acids to be immobilized) in step (3) are labeled with a fluorescent labeling substance, hybridized microbeads can be recognized using a flow cytometer to monitored the degree of washing and the yield. After confirming that nonspecifically hybridized target nucleic acid fragments each having an attached single-stranded tag are detached from the microbeads by washing, covalent bonds are formed using a covalent reaction between the single-stranded tags attached to the target nucleic acid fragments and the anti-tags for immobilization onto microbeads. Although there is no specific limitation concerning the covalent reaction, for example, a DNA ligase such as T4 DNA ligase or *Escherichia coli* DNA ligase can be preferably used. The efficiency of a reaction with a DNA ligase may be increased in some cases by allowing a DNA polymerase to act in the presence of dATP, dGTP, dCTP and dTTP prior to the reaction with the DNA ligase. Thus, such a reaction may optionally be conducted. For example, T4 DNA polymerase can be preferably used although there is no specific limitation concerning the DNA polymerase to be used. Confirmation of processing steps and verification of reaction efficiencies are possible using a detectable labeling substance as an index in steps of a covalent reaction such as a ligation reaction with a DNA ligase, an extension reaction with a DNA polymerase, and inactivation of enzymes and washing associated with the reactions.

(5) Step of isolating microbeads each having a specifically bound target nucleic acid
The target nucleic acids are labeled with the detectable labeling substance and the labeling substance capable of binding to a solid phase. After washing, microbeads are isolated utilizing the labeling substance capable of binding to a solid phase. The labeling substance capable of binding to a solid phase may be any one that is capable of directly or indirectly binding to a solid phase other than the microbead. There is no specific limitation concerning the bond. The bond is exemplified by a covalent bond; a hydrogen bond, an ionic bond, an electrostatic bond or an intermolecular interaction. For example, if biotin is used, a solid phase (e.g., a magnetic bead) having attached avidin can be preferably used as a substrate although it is not intended to limit the present invention. Microbeads each having a specifically bound target nucleic acid and bound to a substrate are washed, and the microbeads each having a specifically bound target nucleic acid are then released from the substrate. Although there is no specific limitation concerning the releasing method, a method of excision using a restriction enzyme utilizing recognition sites for the restriction enzyme in the target nucleic acids can be preferably used. It is possible to rapidly and conveniently prepare microbeads each having a specifically bound target nucleic acid without the use of an instrument such as a cell sorter by isolating the microbeads using a labeling substance that binds to a substrate.

### Examples

The following examples further illustrate the present invention in detail but are not to be construed to limit the scope thereof.

Among the procedures described herein, basic procedures including preparation of plasmid DNAs and restriction enzyme digestion were carried out as described in Sambrook et al., Molecular Cloning - A Laboratory Manual - 3rd ed., Cold Spring Harbor Laboratory Press (2001). Unless otherwise stated, *Escherichia coli* TOP10 was used as a host for the construction of plasmids using *Escherichia coli.* Transformed *Escherichia coli* cells were cultured aerobically at 37°C using LB medium (1% Tryptone, 0.5% yeast extract, 0.5% NaCl, pH 7.0) containing 30 µg/ml of chloramphenicol or LB-chloramphenicol plate prepared by adding agar at concentration of 1.5% to the above-mentioned medium and solidifying the resulting mixture.

### Example 1: Preparation of sample

HepG2 (ATCC HB-8065) was cultured in RPMI 1640 medium supplemented with 10% fetal bovine serum (FBS) for seven days at 37°C in the presence of 5% CO₂. Total RNA was then extracted using TRIzol reagent (Gibco) from the collected cells. polyA RNAs were purified from the total RNA using Oligotex Super (Takara Bio).

### Example 2: Preparation of tag library

A tag vector pMBS1 as described in US 2004/0002104 was digested with BamHI and BbsI (both from New England Biolabs), and dephosphorylated with calf intestine alkaline phosphatase (CIAP, Takara Bio).

A reverse transcription reaction was carried out using 1 µg of the cell-derived polyA RNAs as templates, a mixture of equal amounts of three biotinylated primers of SEQ ID NOS:1-3, 5-methyl-dCTP, dATP, dGTP and dTTP as substrates as well as M-MLV RTase (Takara Bio). Next, 2nd strand synthesis was carried out using 5-methyl-dCTP, dATP, dGTP and dTTP as substrates, RNase H, *E. coli* DNA ligase and *E. coli* DNA polymerase I (all from Takara Bio), and the synthesized double-stranded cDNAs were purified. The double-stranded cDNAs were digested with DpnII (NEB), and an adaptor DNA having an MmeI site in an internal portion was ligated thereto using T4 DNA ligase (Takara Bio). The adaptor DNA was prepared by annealing equal amounts of oligonucleotides of SEQ ID NOS:4 and 5 to each other.

The DNAs were bound to 1.5 mg of Dynabeads M-280 streptavidin (magnetic streptavidin beads, Dynal), allowed to stand in MPC (Dynal), and a supernatant was then removed. The streptavidin beads were washed in 10 mM Tris-HCl (pH 8) and 1 mM EDTA, and subjected to digestion with MmeI (NEB) for excision from the streptavidin beads. The solution of the DNAs excised from the streptavidin beads was reacted with shrimp alkaline phosphatase (USB), and purified after inactivating the shrimp alkaline phosphatase. An adaptor DNA having an SfaNI site in an internal portion was ligated to the DNA fragments using T4 DNA ligase (Takara Bio). The adaptor DNA was prepared by annealing an oligonucleotide of SEQ ID NO:6 to each one of sixteen oligonucleotides of SEQ ID NOS:7-22.

Next, a reaction with T7 exonuclease (NEB) was carried out, and the T7 exonuclease was then inactivated. A PCR was carried out using the DNAs as templates, as well as a FAM-labeled oligonucleotide of SEQ ID NO:23 and a FAM-labeled oligonucleotide of SEQ ID NO:24 as primers. Pfu Turbo (Stratagene), and 5-methyl-dCTP, dATP, dGTP and dTTP as substrates were used for the PCR reaction. The PCR products were subjected to phenol extraction, chloroform extraction and ethanol precipitation to purify the DNAs.

The purified PCR products were digested with enzymes DpnII (NEB) and SfaNI (NEB), and the DNAs were purified. The DNAs were subjected to acrylamide gel electrophoresis, a band corresponding to the target nucleic acid fragments of 32 to 33 nucleotides was excised, and the DNA fragments were extracted from the gel.

The cDNA fragments obtained according to the method of the present invention were ligated to the above-mentioned linearized pMBS1 using T4 DNA ligase (Takara Bio). The resulting recombinant plasmids were used to transform *Escherichia coli* TOP10 by electroporation. The number of independent clones was calculated based on the number of colonies formed by inoculation of a part of the transformants on LB-chloramphenicol plates. The remaining transformants were inoculated into LB medium containing chloramphenicol, and plasmid DNAs were purified from the culture, which corresponded to 640,000 clones, using QIAGEN Plasmid Midi Kit (Qiagen) to prepare a tag library.

### Example 3: Preparation of microbeads

A PCR was carried out using the tag library as described in Example 2 above as a template. The PCR reaction was carried out using 5-methyl-dCTP, dATP, dGTP and dTTP as substrates, an oligonucleotide of SEQ ID NO:25 and a 9:1 mixture of a FAM-labeled oligonucleotide of SEQ ID NO:26 and a biotinylated oligonucleotide of SEQ ID NO:26 as primers as well as Ex Taq Hot Start Version (Takara Bio). The PCR products were purified, digested with a restriction enzyme PacI (NEB), and the tag portions were converted into single-stranded ones by allowing T4 DNA polymerase (NEB) to act in the presence of dGTP. The DNAs were then purified.

The target DNA fragments each having an attached single-stranded tag and 7.2 x 10⁷ microbeads each having an attached anti-tag were mixed together and subjected to hybridization in 500 mM NaCl, 10 mM sodium phosphate, 0.01% Tween 20 and 3% dextran sulfate at 69°C for three days. The reactions were carried out in two tubes. The microbeads were washed in 10 mM Tris-HCl (pH 8), 1 mM EDTA and 0.01% Tween 20, and the microbeads in the two tubes were combined in one tube.

Covalent bonds were formed between the target DNA fragments and the anti-tags by allowing T4 DNA ligase to act on the washed microbeads. The mixture was bound to 600 µg of Dynabeads M-280 streptavidin (magnetic streptavidin beads, Dynal). After allowing the microbeads to stand in MPC (Dynal), a supernatant was removed. The microbeads were resuspended in 1 ml of 10 mM Tris-HCl (pH 8), 1 mM EDTA and 0.01% Tween 20 and allowed to stand in MPC (Dynal), and a supernatant was removed. After repeating the above-mentioned washing procedure, only microbeads carrying target DNA fragments each having an attached single-stranded tag were separated.

The separated microbeads were subjected to digestion with DpnII for excision from Dynabeads M-280 streptavidin (magnetic streptavidin beads, Dynal). After allowing Klenow fragment to act on the microbeads in the presence of dGTP, an adaptor DNA was ligated thereto using T4 DNA ligase. The adaptor DNA was prepared by annealing an oligonucleotide of SEQ ID NO:27 to an oligonucleotide of SEQ ID NO:28.

### Example 4: Preparation of microbeads according to conventional method

A PCR was carried out using the tag library prepared in Example 2 as a template. The PCR reaction was carried out using 5-methyl-dCTP, dATP, dGTP and dTTP as substrates, an oligonucleotide of SEQ ID NO:25 and a FAM-labeled oligonucleotide of SEQ ID NO:26 as primers as well as Ex Taq Hot Start Version (Takara Bio). The PCR products were purified, digested with a restriction enzyme PacI (New England Biolabs, NEB), and the tag portions were converted into single-stranded ones by allowing T4 DNA polymerase (NEB) to act in the presence of dGTP. The DNAs were then purified.

The target DNA fragments each having an attached single-stranded tag and 7.2 x 10⁷ microbeads each having an attached anti-tag were mixed together and subjected to hybridization in 500 mM NaCl, 10 mM sodium phosphate, 0.01% Tween 20 and 3% dextran sulfate at 69°C for three days. The reactions were carried out two tubes. The microbeads were washed in 10 mM Tris-HCl (pH 8), 1 mM EDTA and 0.01% Tween 20, and the microbeads in the two tubes were combined in one tube.

Next, 4% of the microbeads with the most intense fluorescence from FAM were sorted using MoFlo cytometer (DacoCytomation) (the first round of sorting).

The sorted microbeads were subjected to digestion with DpnII, Klenow fragment was allowed to act on the microbeads in the presence of.dGTP, and an adaptor DNA was ligated thereto using T4 DNA ligase. The adaptor DNA was prepared by annealing an oligonucleotide of SEQ ID NO:27 to an oligonucleotide of SEQ ID NO:28. Finally, microbeads with fluorescence from FAM were sorted using MoFlo cytometer (the second round of sorting).

### Example 5: MPSS analysis

The sequences of target DNAs immobilized on the microbeads prepared in Examples 3 and 4 were determined using the technique disclosed in Patent Document 2 or Non-patent Document 2, and the numbers of identical sequences were counted. The number of each sequence per one million sequences was calculated as follows: all the counted numbers of identical sequences were added to determined the total number; each counted number of identical sequences was divided by the total number; the quotient was multiplied by one million. The numbers of the respective sequences per one million sequences for the microbeads prepared in Example 3 as well as those for the microbeads prepared in Example 4 were calculated, and the values were compared with each other. The results of the comparisons are shown in Figure 1. Figure 1 is a scatter plot in which the X axis represents the number of sequences obtained using the microbeads prepared in Example 4 according to the conventional method, and the Y axis represents the number of sequences obtained using the microbeads prepared in Example 3 according to the present invention.

The correlation coefficient R² obtained as a result of the comparisons between the two groups of values was 0.98, indicating a very high correlation. When 300,600,000 beads as the working unit according to the method of Brenner et al. were used according to the conventional method in Example 4, five days were required for the procedure following the washing step after the hybridization between target DNA fragments each having an attached single-stranded tag and microbeads each having an attached anti-tag. Furthermore, the required number of working days increased in proportion to the number of beads. On the other hand, the same procedure could be carried out in two days regardless of the number of beads according to the present invention in Example 3. Thus, the working time could be greatly shortened.

As described above, it was demonstrated that, according to the present invention, microbeads could be prepared without sorting more conveniently in a shorter time as compared with the conventional method, and results equivalent to those of the conventional method could be obtained.

The present invention provides a method for conveniently sorting microbeads each having a bound target DNA for preparation of a microbead array.

### Sequence Listing Free Text:

SEQ ID NO:1: Synthetic primer for reverse transcription
SEQ ID NO:2: Synthetic primer for reverse transcription
SEQ ID NO:3: Synthetic primer for reverse transcription
SEQ ID NO:4: Synthetic oligonucleotide for adaptor DNA
SEQ ID NO:5: Synthetic oligonucleotide for adaptor DNA
SEQ ID NO:6: Synthetic oligonucleotide for adaptor DNA
SEQ ID NO:7: Synthetic oligonucleotide for adaptor DNA
SEQ ID NO:8: Synthetic oligonucleotide for adaptor DNA
SEQ ID NO:9: Synthetic oligonucleotide for adaptor DNA
SEQ ID NO:10: Synthetic oligonucleotide for adaptor DNA
SEQ ID NO:11: Synthetic oligonucleotide for adaptor DNA
SEQ ID NO:12: Synthetic oligonucleotide for adaptor DNA
SEQ ID NO:13: Synthetic oligonucleotide for adaptor DNA
SEQ ID NO:14: Synthetic oligonucleotide for adaptor DNA
SEQ ID NO:15: Synthetic oligonucleotide for adaptor DNA
SEQ ID NO:16: Synthetic oligonucleotide for adaptor DNA
SEQ ID NO:17: Synthetic oligonucleotide for adaptor DNA
SEQ ID NO:18: Synthetic oligonucleotide for adaptor DNA
SEQ ID NO:19: Synthetic oligonucleotide for adaptor DNA
SEQ ID NO:20: Synthetic oligonucleotide for adaptor DNA
SEQ ID NO:21: Synthetic oligonucleotide for adaptor DNA
SEQ ID NO:22: Synthetic oligonucleotide for adaptor DNA
SEQ ID NO:23: Synthetic primer to amplify cDNA fragments
SEQ ID NO:24: Synthetic primer to amplify cDNA fragments
SEQ ID NO:25: Synthetic primer to amplify cDNA fragments
SEQ ID NO:26: Synthetic primer to amplify cDNA fragments
SEQ ID NO:27: Synthetic oligonucleotide for adaptor DNA
SEQ ID NO:28: Synthetic oligonucleotide for adaptor DNA

## Claims

1. A method for purifying a microbead having an immobilized nucleic acid as a target, the method comprising:
(a) immobilizing a nucleic acid labeled with at least two labeling substances to a microbead through a covalent bond, wherein at least one of the labeling substances is a detectable labeling substance and another labeling substance is a labeling substance capable of binding to a solid phase that is different from the microbead; and
(b) binding the microbead having the immobilized nucleic acid to the solid phase that is different from the microbead to isolate the bound microbead.

2. The method according to claim 1, wherein the at least two labeling substances are placed on the same molecule of the nucleic acid.

3. The method according to claim 1, wherein the detectable labeling substance is a labeling substance selected from the group consisting of fluorescent substances, chemiluminescent substances, enzymes and radioisotopes.

4. The method according to claim 1, wherein the labeling substance capable of binding to a solid phase that is different from the microbead is a labeling substance selected from the group consisting of biotin, avidin and haptens.

5. A kit for the method for purifying a microbead having an immobilized nucleic acid defined by claim 1.

6. A microbead array which is an array of microbeads each having an immobilized nucleic acid, wherein the nucleic acid as a target immobilized on each microbead consists of molecules having an identical sequence, the nucleic acid is labeled with at least two labeling substances, at least one of the labeling substances is a detectable labeling substance and another labeling substance is a labeling substance capable of binding to a solid phase that is different from the microbead.

7. The microbead array according to claim 6, wherein the at least two labeling substances are placed on the same molecule of the nucleic acid.

8. The microbead array according to claim 6, wherein the detectable labeling substance is a labeling substance selected from the group consisting of fluorescent substances, chemiluminescent substances, enzymes and radioisotopes.

9. The microbead array according to claim 6, wherein the labeling substance capable of binding to a solid phase that is different from the microbead is a labeling substance selected from the group consisting of biotin, avidin and haptens.

10. A kit for preparing the microbead array defined by claim 6.


SEQUENCE LISTING

<110> TAKARA BIO INC.

<120> Method for purifying microbeads

<130> 38-87

<150> JP 2004-106060

<151> 2004-03-31

<160> 28

<170> PatentIn version 3.1

<210> 1

<211> 41

<212> DNA

<213> Artificial Sequence

<220>

<223> Synthetic primer for reverse transcription

<400> 1

gacatgctgc attgagacga ttcttttttt tttttttttt g 41

<210>  2

<211>  41

<212>  DNA

<213>  Artificial Sequence

<220>

<223>  Synthetic primer for reverse transcription

<400>  2
gacatgctgc attgagacga ttcttttttt tttttttttt c          41

<210>  3

<211>  41

<212>  DNA

<213>  Artificial Sequence

<220>

<223>  Synthetic primer for reverse transcription

<400>  3
gacatgctgc attgagacga ttcttttttt tttttttttt a          41

<210>  4

<211>  23

<212>  DNA

<213>  Artificial Sequence

<220>

<223>  Synthetic oligonucleotide for adaptor DNA

<400>  4

cgttcagagt tctacagtcc gac                                              23

<210>  5

<211>  26

<212>  DNA

<213>  Artificial Sequence

<220>

<223>  Synthetic oligonucleotide for adaptor DNA

<400>  5

gatcgtcgga ctgtagaact ctgaac                                           26

<210>  6

<211>  37

<212>  DNA

<213>  Artificial Sequence

<220>

<223>  Synthetic oligonucleotide for adaptor DNA

<400>  6

ggttcagcag gaatgctcaa tgatgctgac ggctgtt                    37

<210>  7

<211>  36

<212>  DNA

<213>  Artificial Sequence

<220>

<223>  Synthetic oligonucleotide for adaptor DNA

<400>  7

agccgtcagc atcattgagc attcctgctg aaccaa                    36

<210>  8

<211>  36

<212>  DNA

<213>  Artificial Sequence

<220>

<223>  Synthetic oligonucleotide for adaptor DNA

<400>  8
agccgtcagc atcattgagc attcctgctg aaccac                    36

<210>  9

<211>  36

<212>  DNA

<213>  Artificial Sequence

<220>

<223>  Synthetic oligonucleotide for adaptor DNA

<400>  9
agccgtcagc atcattgagc attcctgctg aaccag                    36

<210>  10

<211>  36

<212>  DNA
<213>  Artificial Sequence

<220>
<223>  Synthetic oligonucleotide for adaptor DNA

<400>  10
agccgtcagc atcattgagc attcctgctg aaccat                    36

<210>  11
<211>  36
<212>  DNA
<213>  Artificial Sequence

<220>
<223>  Synthetic oligonucleotide for adaptor DNA

<400>  11
agccgtcagc atcattgagc attcctgctg aaccca                    36

<210>  12
<211>  36
<212>  DNA

<213>  Artificial Sequence

<220>
<223>  Synthetic oligonucleotide for adaptor DNA

<400>  12
agccgtcagc atcattgagc attcctgctg aacccc                    36

<210>  13
<211>  36
<212>  DNA
<213>  Artificial Sequence

<220>
<223>  Synthetic oligonucleotide for adaptor DNA

<400>  13
agccgtcagc atcattgagc attcctgctg aacccg                    36

<210>  14
<211>  36
<212>  DNA
<213>  Artificial Sequence

<220>

<223>  Synthetic oligonucleotide for adaptor DNA

<400>  14
agccgtcagc atcattgagc attcctgctg aaccct                              36

<210>  15
<211>  36
<212>  DNA
<213>  Artificial Sequence

<220>
<223>  Synthetic oligonucleotide for adaptor DNA

<400>  15
agccgtcagc atcattgagc attcctgctg aaccga                              36

<210>  16
<211>  36
<212>  DNA
<213>  Artificial Sequence

<220>

<223>  Synthetic oligonucleotide for adaptor DNA

<400>  16
agccgtcagc atcattgagc attcctgctg aaccgc                              36

<210>  17
<211>  36
<212>  DNA
<213>  Artificial Sequence

<220>

<223>  Synthetic oligonucleotide for adaptor DNA

<400>  17
agccgtcagc atcattgagc attcctgctg aaccgg                              36

<210>  18
<211>  36
<212>  DNA
<213>  Artificial Sequence

<220>

<223>  Synthetic oligonucleotide for adaptor DNA

<400>  18
agccgtcagc atcattgagc attcctgctg aaccgt                    36

<210>  19
<211>  36
<212>  DNA
<213>  Artificial Sequence

<220>
<223>  Synthetic oligonucleotide for adaptor DNA

<400>  19
agccgtcagc atcattgagc attcctgctg aaccta                    36

<210>  20
<211>  36
<212>  DNA
<213>  Artificial Sequence

<220>
<223>  Synthetic oligonucleotide for adaptor DNA

<400> 20
agccgtcagc atcattgagc attcctgctg aacctc 36

<210> 21
<211> 36
<212> DNA
<213> Artificial Sequence

<220>
<223> Synthetic oligonucleotide for adaptor DNA

<400> 21
agccgtcagc atcattgagc attcctgctg aacctg 36

<210> 22
<211> 36
<212> DNA
<213> Artificial Sequence

<220>
<223> Synthetic oligonucleotide for adaptor DNA

<400>  22

agccgtcagc atcattgagc attcctgctg aacctt                              36

<210>  23

<211>  22

<212>  DNA

<213>  Artificial Sequence

<220>

<223>  Synthetic primer to amplify cDNA fragments

<400>  23

cgttcagagt tctacagtcc ga                                             22

<210>  24

<211>  22

<212>  DNA

<213>  Artificial Sequence

<220>

<223>  Synthetic primer to amplify cDNA fragments

<400>  24

agccgtcagc atcattgagc at                                    22

<210>  25

<211>  22

<212>  DNA

<213>  Artificial Sequence

<220>

<223>  Synthetic primer to amplify cDNA  fragments

<400>  25

cgtccagact tctactacct ca                                    22

<210>  26

<211>  22

<212>  DNA

<213>  Artificial Sequence

<220>

<223>  Synthetic primer to amplify cDNA fragments

<400>  26

gtggtcgtat cttgtcagca tc                                    22

<210>  27

<211>  17

<212>  DNA

<213>  Artificial Sequence

<220>

<223>  Synthetic oligonucleotide for adaptor DNA

<400>  27

atcacgagct gccagtc                                                    17

<210>  28

<211>  14

<212>  DNA

<213>  Artificial Sequence

<220>

<223>  Synthetic oligonucleotide for adaptor DNA

<400>  28

gactggcagc tcgt                                                       14